(19) Europäisches Patentamt
European Patent Office
Office européen des brevets

(11) **EP 4 318 337 A1**

(12) **EUROPEAN PATENT APPLICATION**
published in accordance with Art. 153(4) EPC

(43) Date of publication:
**07.02.2024 Bulletin 2024/06**

(21) Application number: **22795649.7**

(22) Date of filing: **20.04.2022**

(51) International Patent Classification (IPC):
**G06N 20/00** (2019.01)    **G06F 30/20** (2020.01)
**G06F 30/27** (2020.01)    **G06F 111/06** (2020.01)

(52) Cooperative Patent Classification (CPC):
**G06F 30/10; G06F 30/20; G06F 30/27;**
**G06N 20/00;** G06F 2111/06

(86) International application number:
**PCT/JP2022/018312**

(87) International publication number:
**WO 2022/230736 (03.11.2022 Gazette 2022/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.04.2021 JP 2021075151**

(71) Applicant: **RESONAC CORPORATION**
**Tokyo 105-7325 (JP)**

(72) Inventor: **HANAOKA Kyohei**
**Tokyo 100-6606 (JP)**

(74) Representative: **Berggren Oy**
**P.O. Box 16**
**Eteläinen Rautatiekatu 10A**
**00101 Helsinki (FI)**

(54) **DESIGN AID DEVICE, DESIGN AID METHOD, AND DESIGN AID PROGRAM**

(57) A design aid device according to an embodiment includes a data acquisition unit configured to acquire performance data including a design parameter group and an observation value of a feature item, a model construction unit configured to construct a predictive model predicting an observation value as a probability distribution, etc. based on the design parameter group, a sampling unit configured to sample a predetermined number of points of objective variable groups using each predictive model, an evaluation value calculation unit configured to convert a vector whose elements are values of respective objective variables included in an objective variable group into a scalar, thereby calculating an evaluation value of at each sampling point, an acquisition function evaluation unit configured to output an acquisition function evaluation value based on a distribution of the evaluation value at each sampling point, a design parameter group acquisition unit configured to acquire a design parameter group by optimization of the acquisition function evaluation value, and an output unit configured to output a design parameter group candidate.

*Fig.2*

EP 4 318 337 A1

**Description**

**Technical Field**

An aspect of the present disclosure relates to a design aid device, a design aid method, and a design aid program.

**Background Art**

Product design using machine learning has been studied. As a field of product design, for example, in design of a functional material, for example, a model estimating properties of a material is constructed by machine learning using learning data including a pair of a raw material mixing ratio and properties for a tested and manufactured material, and properties are predicted for a raw material mixing ratio not tested. By planning a test based on such prediction of properties, it is possible to efficiently optimize parameters such as properties and a raw material mixing ratio of a material, and development efficiency is improved. In addition, Bayesian optimization has been known to be effective as such an optimization method, and a design device outputting a design value using Bayesian optimization has been known.

**Citation List**

**Patent Literature**

Patent Literature 1: Japanese Unexamined Patent Publication No. 2020-52737

**Summary of Invention**

**Technical Problem**

Meanwhile, in development of a product such as a material, in a situation where a plurality of objective variables (features) is given, a plurality of objective variables is optimized to improve a plurality of features changing according to design variables. This is referred to as multi-objective optimization. When there is a trade-off between objective variables, there is a plurality of optimal solutions (Pareto solutions), and one solution cannot be determined. For example, when a target value is set for each objective variable, to obtain an optimal Pareto solution, it is conceivable to take an approach of finding many Pareto solutions and selecting a Pareto solution close to a design target. However, in such an approach, evaluation of a lot of objective functions is required, and a processing load becomes enormous, which is not practical. Such a problem is not limited to material design, and is common to overall product design.

Therefore, the present invention has been made in view of the above-mentioned problems, and an object thereof is to allow optimization of a feature and a design variable of a product included in objective variables with a low load using fewer tests in a manufacturing process of a product, a partly finished product, a semifinished product, a part, or a trial product.

**Solution to Problem**

A design aid device according to an aspect of the present disclosure is a design aid device for obtaining a plurality of design parameters improving a plurality of feature items indicating features of a product, a partly finished product, a semifinished product, a part, or a trial product to be applied to a method of optimizing a design parameter by repeating determination of a design parameter and manufacture of a product, a partly finished product, a semifinished product, a part, or a trial product based on the determined design parameter in design of a product, a partly finished product, a semifinished product, a part, or a trial product manufactured based on a design parameter group including a plurality of design parameters, the design aid device including a data acquisition unit configured to acquire a plurality of pieces of performance data each including the design parameter group and an observation value of each of the plurality of feature items with regard to the manufactured product, partly finished product, semifinished product, part, or trial product, a model construction unit configured to construct, based on the performance data, a predictive model predicting an observation value of the feature item serving as an objective variable as a probability distribution or an approximation or alternative index thereof based on the design parameter group, a sampling unit configured to set a plurality of objective variable groups sampled from a multidimensional probability distribution of observation values obtained from each predictive model as one sampling point, and sample a predetermined number of points of the objective variable groups, an evaluation value calculation unit configured to convert a vector whose dimension is a number of objective variables included in each of the objective variable groups and whose elements are values of the respective objective variables into a scalar through a predetermined operation, thereby calculating an evaluation value of an objective variable group at each sampling point, an acquisition function evaluation unit configured to receive the design parameter group as input and output an acquisition function evaluation value related to improvement of the evaluation value using a predetermined acquisition function based on a distribution of the evaluation value at each sampling point, a design parameter group acquisition unit configured to acquire at least one design parameter group by optimization of the acquisition function evaluation value, and an output unit configured to output the design parameter group acquired by the design parameter group acquisition unit.

A design aid method according to an aspect of the present disclosure is a design aid method in a design aid device for obtaining a plurality of design parameters improving a plurality of feature items indicating features of a product, a partly finished product, a semifinished product, a part, or a trial product to be applied to a method of optimizing a design parameter by repeating determination of a design parameter and manufacture of a product, a partly finished product, a semifinished product, a part, or a trial product based on the determined design parameter in design of a product, a partly finished product, a semifinished product, a part, or a trial product manufactured based on a design parameter group including a plurality of design parameters, the design aid method including a data acquisition step of acquiring a plurality of pieces of performance data each including the design parameter group and an observation value of each of the plurality of feature items with regard to the manufactured product, partly finished product, semifinished product, part, or trial product, a model construction step of constructing, based on the performance data, a predictive model predicting an observation value of the feature item serving as an objective variable as a probability distribution or an approximation or alternative index thereof based on the design parameter group, a sampling step of setting a plurality of objective variable groups sampled from a multidimensional probability distribution of observation values obtained from each predictive model as one sampling point, and sampling a predetermined number of points of the objective variable groups, an evaluation value calculation step of converting a vector whose dimension is a number of objective variables included in the objective variable groups and whose elements are values of the respective objective variables into a scalar through a predetermined operation, thereby calculating an evaluation value of an objective variable group at each sampling point, an acquisition function evaluation step of receiving the design parameter group as input and outputting an acquisition function evaluation value related to improvement of the evaluation value using a predetermined acquisition function based on a distribution of the evaluation value at each sampling point, a design parameter group acquisition step of acquiring at least one design parameter group by optimization of the acquisition function evaluation value, and an output step of outputting the design parameter group acquired by the design parameter group acquisition step.

A design aid program according to an aspect of the present disclosure is a design aid program for causing a computer to function as a design aid device for obtaining a plurality of design parameters improving a plurality of feature items indicating features of a product, a partly finished product, a semifinished product, a part, or a trial product to be applied to a method of optimizing a design parameter by repeating determination of a design parameter and manufacture of a product, a partly finished product, a semifinished product, a part, or a trial product based on the determined design parameter in design of a product, a partly finished product, a semifinished product, a part, or a trial product manufactured based on a design parameter group including a plurality of design parameters, the design aid program causing the computer to realize a data acquisition function of acquiring a plurality of pieces of performance data including the design parameter group and an observation value of each of the plurality of feature items with regard to the manufactured product, partly finished product, semifinished product, part, or trial product, a model construction function of constructing, based on the performance data, a predictive model predicting an observation value of the feature item serving as an objective variable as a probability distribution or an approximation or alternative index thereof based on the design parameter group, a sampling function of setting a plurality of objective variable groups sampled from a multidimensional probability distribution of observation values obtained from each predictive model as one sampling point, and sampling a predetermined number of points of the objective variable groups, an evaluation value calculation function of converting a vector whose dimension is a number of objective variables included in the objective variable groups and whose elements are values of the respective objective variables into a scalar through a predetermined operation, thereby calculating an evaluation value of an objective variable group at each sampling point, an acquisition function evaluation function of receiving the design parameter group as input and outputting an acquisition function evaluation value related to improvement of the evaluation value using a predetermined acquisition function based on a distribution of the evaluation value at each sampling point, a design parameter group acquisition function of acquiring at least one design parameter group by optimization of the acquisition function evaluation value, and an output function of outputting the design parameter group acquired by the design parameter group acquisition function.

According to such an aspect, a predictive model predicting an observation value of a feature item is constructed based on performance data. This predictive model predicts the observation value serving as an objective variable as a probability distribution or an approximation or alternative index thereof, and thus can sample any number of points of objective variable groups based on a multidimensional distribution of observation values obtained from a predictive model of each feature item. By performing a predetermined operation on a vector whose elements are objective variable groups at respective sampling points, it is possible to obtain an evaluation value related to each sampling point expressed by a scalar value. Further, by optimizing an acquisition function evaluation value output using a predetermined acquisition function based on a distribution of evaluation values at respective sampling points, it is possible to obtain a design parameter group suitable for a next test, etc. Therefore, when compared to a general method of directly learning an evaluation value and constructing an acquisition function, a more accurate machine learning model can be obtained, and as a result, the number of tests can be reduced by improving desirability of a design parameter group used in a test, etc.

In a design aid device according to another aspect, the evaluation value calculation unit may calculate the evaluation value including a weighted sum of objective variables included in the objective variable group.

According to such an aspect, by including the weighted sum of objective variables in the evaluation value, it is possible to obtain an evaluation value appropriately representing characteristics of each sampling point. Further, the evaluation value calculation unit may use the objective variable as input and calculate any scalar value calculated by predetermined processing as the evaluation value.

In a design aid device according to another aspect, when a target value is set for each objective variable, the evaluation value calculation unit may calculate the evaluation value further including a difference between a target value and an objective variable having a largest difference from the target value among a plurality of objective variables included in the objective variable group.

According to such an aspect, when optimization is a minimization problem, the evaluation value calculation unit may calculate an evaluation value further including a difference between a target value and an objective variable having a largest numerical value based on the target value among a plurality of objective variables included in the objective variable group. In addition, when optimization is a maximization problem, the evaluation value calculation unit may calculate an evaluation value further including a difference between a target value and an objective variable having a smallest numerical value based on the target value among a plurality of objective variables included in the objective variable group. By optimizing the acquisition function evaluation value obtained based on the evaluation value calculated in this way, it is possible to obtain an effective design parameter group for bringing an objective variable farthest from the target value closer to the target value in one process of optimization.

In a design aid device according to another aspect, the acquisition function evaluation unit may output the acquisition function evaluation value using any one acquisition function among LCB (Lower Confidence Bound), UCB (Upper Confidence Bound), EI (Expected Improvement), and PI (Probability of Improvement).

According to such an aspect, an acquisition function evaluation value suitable for evaluating the design parameter group suitable for improving the evaluation value is output.

In a design aid device according to another aspect, the design parameter group acquisition unit may acquire one design parameter group optimizing the acquisition function evaluation value.

According to such an aspect, it is possible to obtain a design parameter group capable of improving an evaluation value related to a feature item.

In a design aid device according to another aspect, the design parameter group acquisition unit may acquire a plurality of design parameter groups using a predetermined algorithm.

According to such an aspect, it is possible to easily obtain a plurality of design parameter groups used in a next test.

In a design aid device according to another aspect, the predictive model may be a regression model or a classification model receiving the design parameter group as input and outputting a probability distribution of the observation values, and the model construction unit may construct the predictive model by machine learning using the performance data.

According to such an aspect, since the predictive model is constructed as a predetermined regression model or classification model, a predictive model allowing acquisition of a probability distribution of the observation values of the feature items or an approximation or alternative index thereof is obtained.

In a design aid device according to another aspect, the predictive model may be a machine learning model predicting a probability distribution of observation values or an approximation or alternative index thereof using any one of a posterior distribution of predictive values based on Bayesian theory, a distribution of predictive values of a predictor included in an ensemble, a theoretical formula for a prediction interval and a confidence interval of a regression model, Monte Carlo dropout, and a distribution of predictions of a plurality of predictors constructed under different conditions.

According to such an aspect, a predictive model allowing predicting of a probability distribution of the observation values of the feature items or an approximation or alternative index thereof based on the design parameter group is constructed.

**Advantageous Effects of Invention**

According to an aspect of the present disclosure, a feature and a design variable of a product, etc. included in objective variables is allowed to be optimized with a low load using fewer tests in a manufacturing process of a product, a partly finished product, a semifinished product, a part, or a trial product.

**Brief Description of Drawings**

FIG. 1 is a diagram illustrating an outline of a material design process to which a design aid device according to an embodiment is applied.
FIG. 2 is a block diagram illustrating an example of a functional configuration of the design aid device according to the embodiment.
FIG. 3 is a hardware block diagram of the design aid device according to the embodiment.
FIG. 4 is a diagram illustrating an example of a design parameter group related to a manufactured material.
FIG. 5 is a diagram illustrating an example of an observation value related to a manufactured material.
FIG. 6 is a flowchart illustrating a process of optimizing a feature item and a design parameter in material design.
FIG. 7 is a flowchart illustrating an example of content of a design aid method in the design aid device according to the embodiment.
FIG. 8 is a diagram illustrating a configuration of a design aid program.

**Description of Embodiments**

Hereinafter, embodiments of the invention will be described in detail with reference to the accompanying drawings. Note that, in the description of the drawings, the same or equivalent elements are given the same reference numerals, and redundant description will be omitted.

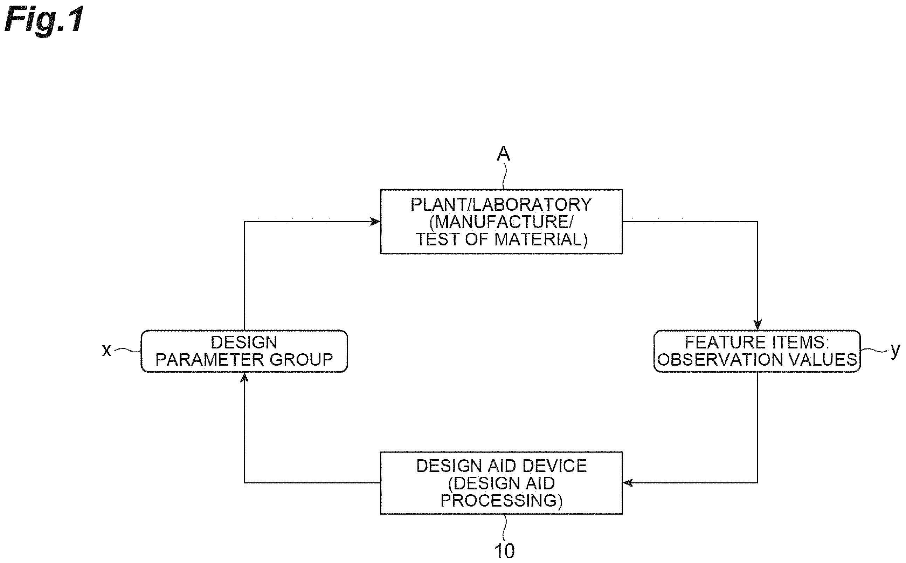

FIG. 1 is a diagram illustrating an outline of a material design process, which is an example of a design process of a product, a partly finished product, a semifinished product, a part, or a trial product to which a design aid device according to an embodiment is applied. Note that, in the following, "the product, the partly finished product, the semifinished product, the part, or the trial product" will be referred to as "product, etc." A design aid device 10 of the present embodiment can be applied to a process of designing a plurality of feature items indicating features of the product etc. and any product, etc. requiring optimization of each feature item. The design aid device 10 can be applied to a method of optimizing a design parameter and an objective variable of a product, etc. by repeating determination of a design parameter and manufacture of a product, a partly finished product, a semifinished product, a part, or a trial product based on the determined design parameter. Specifically, for example, the design aid device 10 can be applied to design of products such as automobiles and drugs, and optimization of a molecular structure of a drug in addition to development and design of materials. In the present embodiment, as described above, design aid processing by the design aid device 10 will be described using an example of material design as an example of design of a product, etc.

As illustrated in FIG. 1, design aid processing by the design aid device 10 is applied to manufacture and a test of a material in a plant, a laboratory A, etc. That is, according to a set design parameter group x, a material is manufactured in the plant, the laboratory A, etc., and observation values y of a plurality of feature items indicating features of the material are acquired based on the manufactured material. Note that, the manufacture and test of the material in the plant and the laboratory A may be simulations. In this case, the design aid device 10 provides the design parameter group x for execution of a next simulation.

The design aid device 10 optimizes a plurality of feature items and design parameters based on performance data including a design parameter group x and observation values y of a plurality of feature items of a material manufactured based on the design parameter group x. Specifically, the design aid device 10 outputs a design parameter group x having a possibility of obtaining more suitable features for next manufacture and test based on a design parameter group x and observation values y related to a manufactured material.

For example, the design aid device 10 of the present embodiment is applied for the purpose of improving a plurality of features by tuning a plurality of design variables in design of a material product. As an example of design of a material product, when a certain material is manufactured by mixing a plurality of polymers and additives, the design aid device 10 uses a design parameter group of a mixing amount of each polymer and additive, etc. as a design variable, and uses observation values of a modulus of elasticity and a coefficient of thermal expansion, which are feature items, as objective variables to tune a design parameter group improving one evaluation value set for a plurality of features.

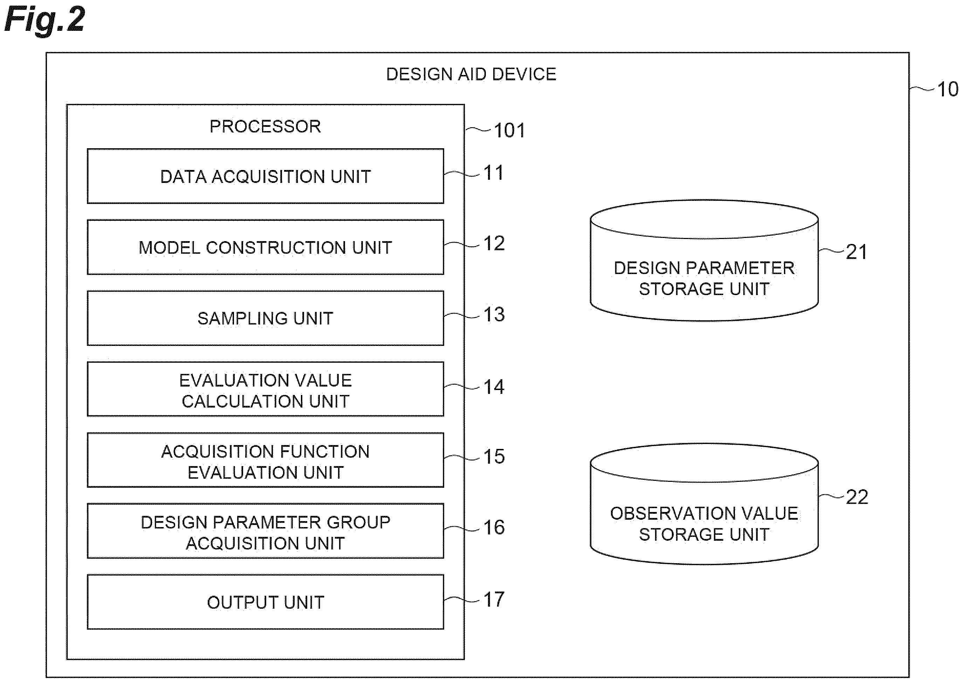

FIG. 2 is a block diagram illustrating an example of a functional configuration of the design aid device according to the embodiment. The design aid device 10 is a device that obtains a plurality of design parameters improving one evaluation value set for a plurality of features indicating a material feature in design of a material manufactured based on a design parameter group including a plurality of design parameters. As illustrated in FIG. 2, the design aid device 10 may include a functional unit configured as a processor 101, a design parameter storage unit 21, and an observation value storage unit 22. Each functional unit will be described later.

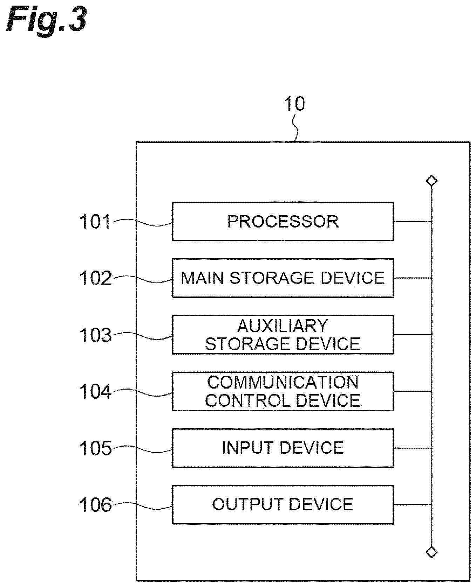

FIG. 3 is a diagram illustrating an example of a hardware configuration of a computer 100 included in the design aid device 10 according to the embodiment. Note that, the computer 100 can be included in the design aid device 10.

As an example, the computer 100 includes a processor 101, a main storage device 102, an auxiliary storage device 103, and a communication control device 104 as hardware components. The computer 100 included in the design aid device 10 may further include an input device 105 such as a keyboard, a touch panel, or a mouse, and an output device 106 such as a display.

Processor 101 is a computing device that executes an operating system and application programs. Examples of processors include a CPU (Central Processing Unit) and a GPU (Graphics Processing Unit), but a type of the processor 101 is not limited thereto. For example, the processor 101 may be a combination of a sensor and a dedicated circuit. The dedicated circuit may be a programmable circuit such as an FPGA (Field-Programmable Gate Array), or may be another type of circuit.

The main storage device 102 is a device that stores a program for implementing the design aid device 10, etc., a calculation result output from the processor 101, etc. The main storage device 102 includes, for example, at least one of a ROM (Read Only Memory) and a RAM (Random Access Memory).

The auxiliary storage device 103 is generally a device that can store a larger amount of data than that of the main storage device 102. For example, the auxiliary storage device 103 includes a nonvolatile storage medium such as a hard disk or a flash memory. The auxiliary storage device 103 stores a design aid program P1 and various data for causing the computer 100 to function as the design aid device 10, etc.

The communication control device 104 is a device that performs data communication with another computer via a communication network. For example, the communication control device 104 includes a network card or a wireless communication module.

Each functional element of the design aid device 10 is realized by reading the corresponding program P1 onto the processor 101 or the main storage device 102 and causing the processor 101 to execute the program. The program P1 includes code for implementing each functional element of the corresponding server. The processor 101 operates the communication control device 104 according to the program P1, and executes reading and writing of data in the main storage device 102 or the auxiliary storage device 103. Through such processing, each functional element of the corresponding server is realized.

The program P1 may be provided after being permanently recorded on a tangible recording medium such as a CD-ROM, a DVD-ROM, or a semiconductor memory. Alternatively, at least one of these programs may be provided via a communication network as a data signal superimposed on a carrier wave.

Referring again to FIG. 2, the design aid device 10 includes a data acquisition unit 11, a model construction unit 12, a sampling unit 13, an evaluation value calculation unit 14, an acquisition function evaluation unit 15, a design parameter group acquisition unit 16, and an output unit 17. The design parameter storage unit 21 and the observation value storage unit 22 may be configured in the design aid device 10 as illustrated in FIG. 2, or may be configured as other devices that can be accessed from the design aid device 10.

The data acquisition unit 11 acquires a plurality of pieces of performance data related to a manufactured material. The performance data includes a pair of a design parameter group and an observation value of each of a plurality of feature items. The design parameter storage unit 21 is a storage means that stores a design parameter group in the performance data, and may be included in, for example, the main storage device 102, the auxiliary storage device 103, etc. The observation value storage unit 22 is a storage means that stores an observation value in the performance data.

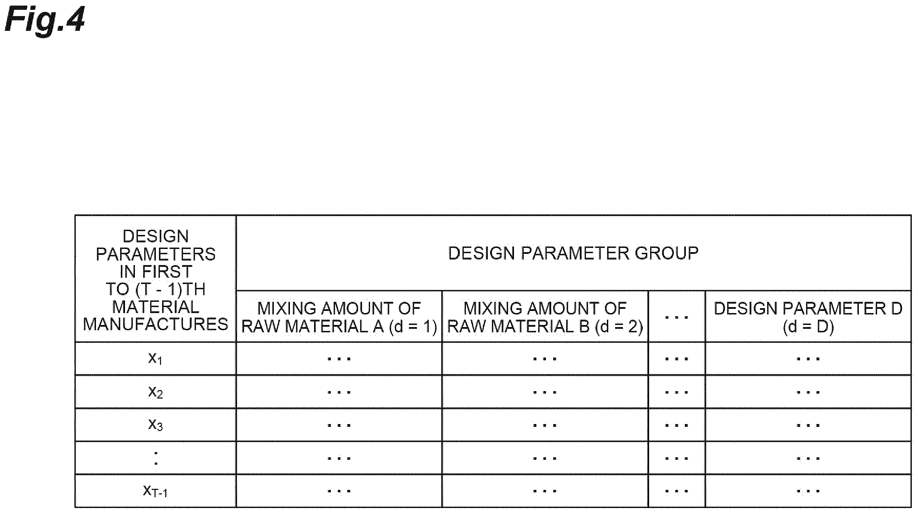

FIG. 4 is a diagram illustrating an example of a design parameter group stored in the design parameter storage unit 21. As illustrated in FIG. 4, the design parameter storage unit 21 stores a design parameter group $x_t$ in each of first ($t = 1$) to ($T - 1$)th material manufactures ($t = T - 1$). The design parameter group x may include, as an example, a mixing amount of a raw material A, a mixing amount of a raw material B, a design parameter D, etc., and can be included in vector data having the number of dimensions corresponding to the number D of design parameters. In addition to the illustrated design parameters, the design parameters may be, for example, non-vector data of a molecular structure, an image, and the like. Furthermore, when dealing with a problem of selecting an optimal molecule from a plurality of molecule types, the design parameters may be data indicating options among the plurality of molecules.

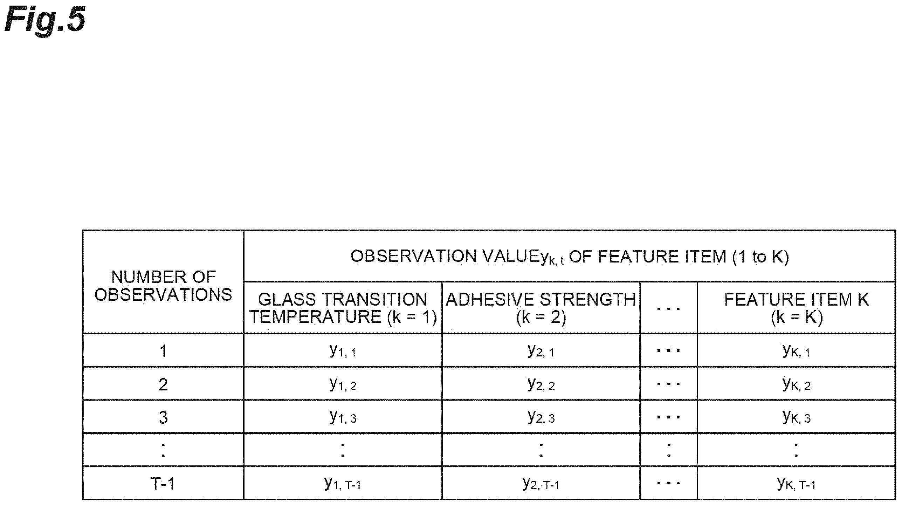

FIG. 5 is a diagram illustrating an example of an observation value y stored in the observation value storage unit 22. As illustrated in FIG. 5, the observation value storage unit 22 stores an observation value $y_{k,t}$ of each of a plurality of feature items k ($k = 1$ to K) indicating features of materials manufactured in the first ($t = 1$) to ($T - 1$)th material manufactures ($t = T - 1$). The feature items k may include, as an example, glass transition temperature, adhesive strength, and feature item K. A pair of the design parameter group $x_t$ and the observation value $y_{k,t}$ is included in performance data.

The design aid device 10 obtains a Tth design parameter group $x_T$ improving an observation value of each feature item based on performance data in the first ($t = 1$) to ($T - 1$)th material manufactures ($t = T - 1$).

The model construction unit 12 constructs a predictive model based on performance data. The predictive model is a model predicting an observation value $y_k$ of a feature item k serving as an objective variable as a probability distribution or an approximation or alternative index thereof based on a design parameter group x. It is sufficient that a model included in the predictive model is a model capable of predicting the observation value $y_k$ as a probability distribution or an approximation or alternative index thereof, and a type thereof is not limited.

For example, the predictive model may be a regression model that takes the design parameter x as an input and outputs the probability distribution of the observation value $y_k$. When the predictive model is a regression model, for example, the predictive model may include any one of regression models such as linear regression, PLS regression, Gaussian process regression, random forest, and neural network. The model construction unit 12 may construct a predictive model according to a well-known machine learning method using performance data. The model construction unit 12 may construct a predictive model according to a machine learning method of applying performance data to a predictive model and updating a parameter of the predictive model.

In a predictive model constructed as Gaussian process regression, a probability distribution of observation values is predicted by inputting, to the model, a design parameter group x in performance data included in an explanatory variable of training data, an observation value y included in an objective variable, and a design parameter x to be predicted.

In addition, the predictive model may be a machine learning model predicting a probability distribution of observation values or an approximation or alternative index thereof using any one of a posterior distribution of predictive values based on Bayesian theory, a distribution of predictive values of a predictor included in an ensemble, a theoretical formula for a prediction interval and a confidence interval of a regression model, a distribution obtained by Monte Carlo dropout, and a distribution of predictions of a plurality of predictors constructed under different conditions.

Prediction of a probability distribution of observation values or an alternative index thereof can be obtained by a model-specific method. The probability distribution of the observation values or the approximation or alternative index thereof can be obtained based on a posterior distribution of predictive values in the case of Gaussian process regression and a Bayesian neural network, based on a distribution of predictions of the predictor included in the ensemble in the case of a random forest, based on a prediction interval and a confidence interval in the case of linear regression, and based on Monte Carlo dropout in the case of a neural network. However, a method of calculating a distribution of observation values or an alternative index thereof for each machine learning model is not limited to the above method.

In addition, any model may be extended to a model that can predict a probability distribution of observation values or an alternative index thereof. For example, a model, which constructs a plurality of data sets using a bootstrap method, etc. and uses a distribution of predictive values of each model obtained by constructing a predictive model for each of the data sets as an alternative index of a probability distribution of observation values, is given as an example. However, a method of extending the machine learning model to a model that can predict a probability distribution of observation values or an alternative index thereof is not limited to the above method.

Furthermore, the model construction unit 12 may tune hyperparameters of the predictive model using a well-known hyperparameter tuning method. That is, the model construction unit 12 may update the hyperparameters of the predictive model by maximum likelihood estimation using a vector representing a design parameter group x, which is an explanatory variable in performance data, and an observation value y, which is an objective variable.

Further, the predictive model may be constructed using a classification model. When the predictive model is a classification model, the model construction unit 12 can construct the predictive model using a machine learning method capable of evaluating a well-known probability distribution using performance data.

In this way, when the model construction unit 12 constructs a predictive model using a predetermined regression model or classification model, it becomes possible to obtain a probability distribution of observation values of feature items based on any design parameter group x.

In addition, the predictive model may be a single task model predicting an observation value of one feature item as a probability distribution or an approximation or alternative index thereof, or a multi-task model predicting observation values of a plurality of feature items as a probability distribution or an approximation or alternative index thereof. In this way, by constructing a predictive model using a multi-task model or a single-task model appropriately configured according to properties of feature items, it is possible to improve accuracy of prediction of observation values by the predictive model.

The sampling unit 13 samples a predetermined number of objective variable groups by using a plurality of objective variable groups sampled from a multidimensional probability distribution of observation values obtained from each predictive model as one sampling point.

Specifically, for example, when an observation value $y_k$ (k = 1 to K) serving as an objective variable follows a multidimensional normal distribution based on a predictive model and there is no correlation therebetween, the observation value is expressed as follows.

$y_k$ to $N(m_k(x), c_k(x)^2)$

In such a case, the sampling unit 13 samples a plurality of $y_k$ (k = 1 to K) from the probability distribution of the objective variable $y_k$ based on one design parameter group x.

More specifically, the sampling unit 13 samples each of objective variable groups $y_{k,n}$ (k = 1 to K, and n = 1 to N) as an nth (n: 1 to N) sampling point. As described below, the objective variable group $y_n$ at the nth sampling point forms a vector whose dimension is the number of objective variables included in the objective variable group and whose elements are values of the respective objective variables.

$$y_n = [y_{1,n}, y_{2,n}, \ldots, y_{k,n}, \ldots, y_{K,n}]$$

Then, the sampling unit 13 obtains an objective variable group set Y corresponding to N sampling points, which is a predetermined number of points.

$$Y = [y_1, y_2, \ldots, y_n, \ldots, y_N]$$

Each of objective variable groups $y_1$, $y_2$, ..., $y_n$, ..., $y_N$ is included in a vector.

Note that, in the above example, even though it is assumed that the observation value $y_k$ serving as an objective variable follows a normal distribution and there is no correlation therebetween, there may be a correlation, and the observation value is not limited to follow the normal distribution and may follow another probability distribution. Further, even though sampling by the sampling unit 13 has been described using an example in which sampling is performed for each objective variable, sampling is not limited to such an example, and, for example, the sampling unit 13 may collectively sample objective variable groups based on a multidimensional normal distribution of objective variables defined by a predictive model of each feature item.

Further, the sampling unit 13 may obtain a sample of an objective variable group $y_{k,n}$ based on a sampling point from a standard normal distribution sampled and stored in advance. Specifically, the sampling unit 13 can sample sampling points $y\_std_{k,n}$ (n = 1 to N) in advance from a standard normal distribution, which is a normal distribution having a mean of 0 and a variance of 1, and obtain a sample of the objective variable group $y_{k,n}$ using the following conversion formula.

$$y_{k,n} = y\_std_{k,n} * \sigma_k(x) + m_k(x)$$

Note that, in the design aid device 10 mounted in a computer, the sampling unit 13 may collectively perform sampling at N points, or may collectively perform sampling corresponding to each of a plurality of design parameter groups x.

The evaluation value calculation unit 14 calculates an evaluation value of an objective variable group corresponding to one design parameter group x and one sampling point. Specifically, as mentioned above, since an objective variable group at one sampling point forms a vector whose dimension is the number of objective variables included in the objective variable group and whose elements are values of the respective objective variables, the evaluation value calculation unit 14 calculates an evaluation value at each sampling point by converting the vector representing the objective variable group into a scalar through a predetermined operation. More generally, the evaluation value at each sampling point may be any scalar value calculated by a predetermined process using an objective variable as input.

As an example, the evaluation value calculation unit 14 calculates an evaluation value $v_n$ using a scalarizing function SF as illustrated in the following Equation (1).

$$v_n = SF(y_n) \ \ldots \ (1)$$

The evaluation value $v_n$ forms a scalar value. The scalarizing function SF may include a term for calculation of a weighted sum of objective variables included in the objective variable group $y_n$. In addition, when a target value is set for each objective variable, and when optimization is a minimization problem, the scalarizing function SF may further include a term including a difference between a target value and an objective variable having a largest numerical value based on the target value among a plurality of objective variables included in the objective variable group. In addition, when optimization is a maximization problem, the scalarizing function SF may further include a term including a difference between a target value and an objective variable having a smallest numerical value based on the target value among a plurality of objective variables included in the objective variable group.

For example, when a target value is set for each objective variable and optimization is a minimization problem, the evaluation value calculation unit 14 may calculate the evaluation value $v_n$ using the following Equation (2).

$$v_n = SF(y_n) = \sum_{k=1}^{K}(y_{k,n} * w_k * \rho) + \max_k((y_{k,n} - g_k) * w_k) \ \ldots (2)$$

In Equation (2), a first term is a term for calculation of a weighted sum of objective variables included in the objective variable group $y_n$, $w_k$ is a positive weight for the objective variable $y_k$ and is used to adjust a difference in scale between objective variables, and $\rho$ is any constant for adjustment of balance between the first term and a second term. In addition, in Equation (2), the second term is a term including a difference between a target value and an objective variable having a largest difference from the target value among a plurality of objective variables included in the objective variable group $y_n$, and $g_k$ is a target value of the objective variable $y_k$.

As illustrated in the first term of Equation (2), when the evaluation value $v_n$ includes the weighted sum of the objective variables included in the objective variable group $y_n$, it is possible to promote each objective variable to approach a Pareto solution in a process of Bayesian optimization using the evaluation value $v_n$ calculated in this way. In addition, as illustrated in the second term of Equation (2), when the evaluation value $v_n$ includes a term including a difference between a target value and an objective variable having a largest difference from the target value among a plurality of objective variables included in the objective variable group $y_n$, it is possible to promote the objective variable to approach the target value in a process of Bayesian optimization.

The evaluation value calculation unit 14 can obtain an evaluation value set V including first to Nth evaluation values $v_n$ by calculating the evaluation value $v_n$ of each of objective variable groups $y_n$ at first to Nth sampling points.

$$V = [v_1, v_2, \ldots, v_n, \ldots, v_N]$$

As mentioned above, evaluation values $v_1, v_2, \ldots, v_n, \ldots, v_n$ each form a scalar value.

Note that, in the design aid device 10 mounted in the computer, the evaluation value calculation unit 14 may collectively calculate the evaluation values $v_1, v_2, \ldots, v_n, \ldots, v_N$ corresponding to the N sampling points, respectively.

The acquisition function evaluation unit 15 receives the design parameter group as input and outputs an acquisition function evaluation value related to improvement of the evaluation value using a predetermined acquisition function based on a distribution of an evaluation value at each sampling point.

For example, the acquisition function evaluation unit 15 may output the acquisition function evaluation value using a well-known acquisition function such as LCB (Lower Confidence Bound). LCB is used when minimizing output of a function, and a suitable design parameter group x can be obtained by minimizing a value of LCB.

When an acquisition function is constructed using LCB, the acquisition function evaluation unit 15 defines and constructs an acquisition function evaluation value A(x) as illustrated in the following Equation (3).

$$A(x) = mv(x) - a\sigma v(x) \ldots (3)$$

The acquisition function evaluation unit 15 evaluates and acquires an average $mv(x)$ and a standard deviation $\sigma v(x)$ based on a distribution of evaluation values $v_n$ included in the evaluation value set V, and outputs an acquisition function evaluation value using the acquisition function illustrated in Equation (3). "a" in Equation (3) is any parameter. Equation (3) of the above acquisition function represents a lower limit of a confidence interval when it is assumed that an observation value of $v_n$ in a next test follows a normal distribution when the design parameter group x is used as a parameter.

Furthermore, the acquisition function evaluation unit 15 may output the acquisition function evaluation value A(x) using well-known functions such as UCB (Upper Confidence Bound), EI (Expected Improvement), and PI (Probability of Improvement).

The design parameter group acquisition unit 16 acquires at least one design parameter group by optimizing the acquisition function evaluation value A(x) output by the acquisition function evaluation unit 15.

As an example, the design parameter group acquisition unit 16 may acquire at least one design parameter group x optimizing output of an acquisition function. Specifically, the design parameter group acquisition unit 16 performs optimization using the acquisition function evaluation value A(x) output by the acquisition function evaluation unit 15 as an objective variable, and acquires the design parameter group x as an optimal solution.

Further, as an example, the design parameter group acquisition unit 16 may acquire a plurality of design parameter groups using a predetermined algorithm. Specifically, the design parameter group acquisition unit 16 may acquire a plurality of design parameter groups by applying a batch Bayesian optimization method to an acquisition function. The batch Bayesian optimization method may be, for example, a method such as Local Penalization, but the method is not limited.

The output unit 17 outputs the design parameter groups acquired by the design parameter group acquisition unit 16. That is, the output unit 17 outputs the design parameter groups obtained based on performance data in the first (t = 1) to (T - 1)th material manufactures (t = T - 1) as a design parameter group $x_T$ for a Tth material manufacture.

In addition, when a plurality of design parameter groups is acquired by the design parameter group acquisition unit 16, the output unit 17 outputs acquired design parameter groups as design parameter groups for N material manufactures after a material manufacture subsequent to the (T - 1)th material manufacture. Design parameter groups for a plurality of material manufactures may be used for simultaneous tests and material manufactures.

Even though an aspect of output is not limited, for example, the output unit 17 outputs a design parameter group candidate by causing a predetermined display device to display the design parameter group candidate or causing a predetermined storage means to store the design parameter group candidate.

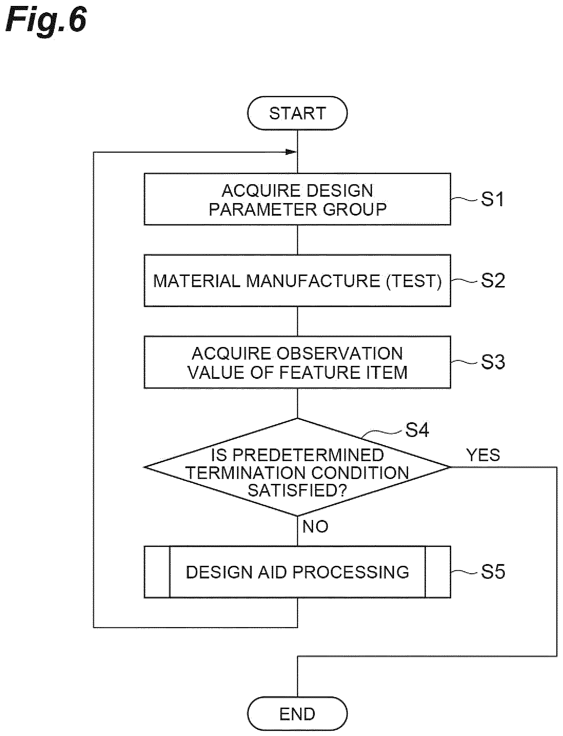

FIG. 6 is a flowchart illustrating a process of optimizing a feature item and a design parameter group in material design.

In step S1, a design parameter group is acquired. The design parameter group acquired here is for initial material manufacture (test), and may be an arbitrarily set design parameter group, or a design parameter group set based on a previously conducted test, etc.

In step S2, material manufacture is performed. In step S3, an observation value of a feature item of the manufactured material is acquired. A pair of the design parameter groups serving as a manufacturing condition in step S2 and the observation value of each feature item acquired in step S3 is included in performance data.

In step S4, it is determined whether a predetermined termination condition is satisfied. The predetermined termination condition is a condition for optimization of the design parameter group and the observation value of the feature item, and may be arbitrarily set. For example, the termination condition for optimization may be reaching a predetermined number of manufactures (tests) and acquisitions of observation values, reaching a target value of the observation value, convergence of optimization, etc. When it is determined that the predetermined termination condition is satisfied, the optimization process is terminated. When it is not determined that the predetermined termination condition is satisfied, the process proceeds to step S5.

In step S5, design aid processing is performed by the design aid device 10. Design aid processing is a process of outputting a design parameter group for next material manufacture. The process then returns to step S1 again.

Note that, in a first cycle of processing cycles including steps S1 to S5, when a plurality of pairs of design parameter groups and observation values of feature items are obtained as initial data, processing of steps S1 to S4 is omitted. When initial data cannot be obtained, in step S1, for example, a design parameter group obtained by any method such as experimental design or random search is acquired. In second and subsequent processing cycles, in step S1, the design parameter group output in step S5 is acquired.

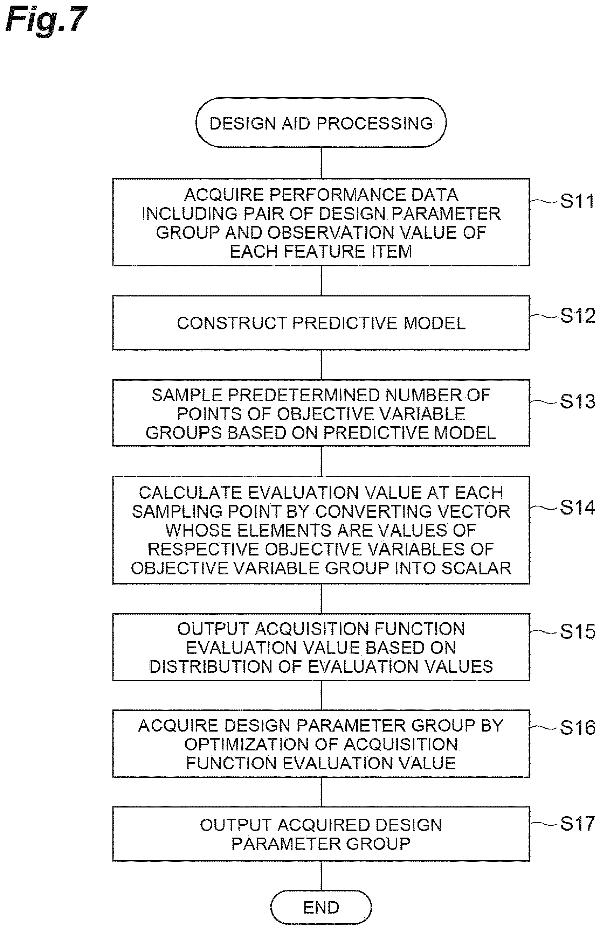

FIG. 7 is a flowchart illustrating an example of content of a design aid method in the design aid device 10 according to an embodiment, and illustrates processing of step S5 of FIG. 6. The design aid method is executed by reading the design aid program P1 in the processor 101 and executing the program to realize each of the functional units 11 to 17.

In step S11, the data acquisition unit 11 acquires a plurality of pieces of performance data related to a manufactured material. The performance data includes a pair of a design parameter group and an observation value of each feature item.

In step S12, the model construction unit 12 constructs a predictive model based on the performance data.

In step S13, the sampling unit 13 sets a plurality of objective variable groups sampled from a multidimensional probability distribution of observation values obtained from each predictive model based on one design parameter group x as one sampling point based on the predictive model, and samples a predetermined number of points of objective variable groups.

In step S14, the evaluation value calculation unit 14 calculates an evaluation value at each sampling point by converting a vector whose elements are values of respective objective variables of the objective variable group into a scalar through a predetermined operation.

In step S15, the acquisition function evaluation unit 15 outputs a predetermined acquisition function evaluation value based on a distribution of an evaluation value at each sampling point.

In step S16, the design parameter group acquisition unit 16 acquires at least one design parameter group by optimizing an acquisition function evaluation value obtained by the acquisition function evaluation unit 15 in step S15.

In step S17, the output unit 17 outputs the design parameter group acquired by the design parameter group acquisition unit 16 in step S16 as a design parameter group for next material manufacture (step S1).

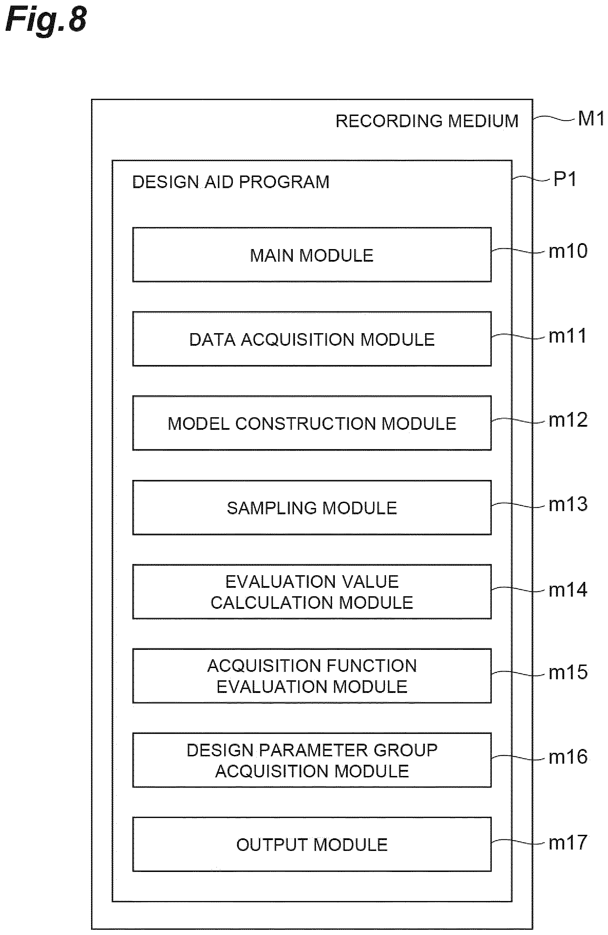

Next, a design aid program for causing a computer to function as the design aid device 10 of the present embodiment will be described. FIG. 8 is a diagram illustrating a configuration of the design aid program.

The design aid program P1 includes a main module m10 that comprehensively controls design aid processing in the design aid device 10, a data acquisition module m11, a model construction module m12, a sampling module m13, an evaluation value calculation module m14, an acquisition function evaluation module m15, a design parameter group acquisition module m16, and an output module m17. Further, each function for each of the data acquisition unit 11, the model construction unit 12, the sampling unit 13, the evaluation value calculation unit 14, the acquisition function evaluation unit 15, the design parameter group acquisition unit 16, and the output unit 17 is realized by each of the modules m11 to m17.

Note that, the design aid program P1 may be transmitted via a transmission medium such as a communication line, or may be stored in a recording medium M1 as illustrated in FIG. 8.

According to the design aid device 10, the design aid method, and the design aid program P1 of the present embodiment described above, a predictive model predicting an observation value of a feature item is constructed based on performance data. This predictive model predicts the observation value serving as an objective variable as a probability distribution or an approximation or alternative index thereof, and thus can sample any number of points of objective variable groups based on a multidimensional distribution of observation values obtained from a predictive model of each feature item. By performing a predetermined operation on a vector whose elements are objective variable groups at respective sampling points, it is possible to obtain an evaluation value related to each sampling point expressed by a scalar value. Further, by optimizing an acquisition function evaluation value output using a predetermined acquisition function based on a distribution of evaluation values at respective sampling points, it is possible to obtain a design parameter group suitable for a next test, etc. Therefore, when compared to a general method of directly learning an evaluation value and constructing an acquisition function, a more accurate machine learning model can be obtained, and as a result, the number of tests can be reduced by improving desirability of a design parameter group used in a test, etc.

The invention has been described above in detail based on the embodiments thereof. However, the invention is not limited to the above embodiments. The invention can be variously modified without departing from the gist thereof.

Reference Signs List

P1: design aid program, m10: main module, m11: data acquisition module, m12: model construction module, m13: sampling module, m14: evaluation value calculation module, m15: acquisition function evaluation module, m16: design parameter group acquisition module, m17: output module, 10: design aid device, 11: data acquisition unit, 12: model construction unit, 13: sampling unit, 14: evaluation value calculation unit, 15: acquisition function evaluation unit, 16: design parameter group acquisition unit, 17: output unit, 21: design parameter storage unit, 22: observation value storage unit.

**Claims**

1. A design aid device for obtaining a plurality of design parameters improving a plurality of feature items indicating features of a product, a partly finished product, a semifinished product, a part, or a trial product to be applied to a method of optimizing a design parameter by repeating determination of a design parameter and manufacture of a product, a partly finished product, a semifinished product, a part, or a trial product based on the determined design parameter in design of a product, a partly finished product, a semifinished product, a part, or a trial product manufactured based on a design parameter group including a plurality of design parameters, the design aid device comprising:

   a data acquisition unit configured to acquire a plurality of pieces of performance data each including the design parameter group and an observation value of each of the plurality of feature items with regard to the manufactured product, partly finished product, semifinished product, part, or trial product;
   a model construction unit configured to construct, based on the performance data, a predictive model predicting an observation value of the feature item serving as an objective variable as a probability distribution or an approximation or alternative index thereof based on the design parameter group;
   a sampling unit configured to set a plurality of objective variable groups sampled from a multidimensional probability distribution of observation values obtained from each predictive model as one sampling point, and sample a predetermined number of points of the objective variable groups;
   an evaluation value calculation unit configured to convert a vector whose dimension is a number of objective variables included in each of the objective variable groups and whose elements are values of the respective objective variables into a scalar through a predetermined operation, thereby calculating an evaluation value of an objective variable group at each sampling point;
   an acquisition function evaluation unit configured to receive the design parameter group as input and output an acquisition function evaluation value related to improvement of the evaluation value using a predetermined acquisition function based on a distribution of the evaluation value at each sampling point;
   a design parameter group acquisition unit configured to acquire at least one design parameter group by optimization of the acquisition function evaluation value; and
   an output unit configured to output the design parameter group acquired by the design parameter group acquisition unit.

2. The design aid device according to claim 1, wherein the evaluation value calculation unit calculates the evaluation value including a weighted sum of objective variables included in the objective variable group.

3. The design aid device according to claim 2, wherein, when a target value is set for each objective variable, the evaluation value calculation unit calculates the evaluation value further including a difference between a target value and an objective variable having a largest difference from the target value among a plurality of objective variables included in the objective variable group.

4. The design aid device according to any one of claims 1 to 3, wherein the acquisition function evaluation unit outputs the acquisition function evaluation value using any one acquisition function among LCB (Lower Confidence Bound), UCB (Upper Confidence Bound), EI (Expected Improvement), and PI (Probability of Improvement).

5. The design aid device according to any one of claims 1 to 4, wherein the design parameter group acquisition unit acquires one design parameter group optimizing the acquisition function evaluation value.

6. The design aid device according to any one of claims 1 to 4, wherein the design parameter group acquisition unit acquires a plurality of design parameter groups using a predetermined algorithm.

7. The design aid device according to any one of claims 1 to 6,

wherein the predictive model is a regression model or a classification model receiving the design parameter group as input and outputting a probability distribution of the observation values, and
the model construction unit constructs the predictive model by machine learning using the performance data.

8. The design aid device according to claim 7, wherein the predictive model is a machine learning model predicting a probability distribution of observation values or an approximation or alternative index thereof using any one of a posterior distribution of predictive values based on Bayesian theory, a distribution of predictive values of a predictor included in an ensemble, a theoretical formula for a prediction interval and a confidence interval of a regression model, Monte Carlo dropout, and a distribution of predictions of a plurality of predictors constructed under different conditions.

9. A design aid method in a design aid device for obtaining a plurality of design parameters improving a plurality of feature items indicating features of a product, a partly finished product, a semifinished product, a part, or a trial product to be applied to a method of optimizing a design parameter by repeating determination of a design parameter and manufacture of a product, a partly finished product, a semifinished product, a part, or a trial product based on the determined design parameter in design of a product, a partly finished product, a semifinished product, a part, or a trial product manufactured based on a design parameter group including a plurality of design parameters, the design aid method comprising:

a data acquisition step of acquiring a plurality of pieces of performance data including the design parameter group and an observation value of each of the plurality of feature items with regard to the manufactured product, partly finished product, semifinished product, part, or trial product;
a model construction step of constructing, based on the performance data, a predictive model predicting an observation value of the feature item serving as an objective variable as a probability distribution or an approximation or alternative index thereof based on the design parameter group;
a sampling step of setting a plurality of objective variable groups sampled from a multidimensional probability distribution of observation values obtained from each predictive model as one sampling point, and sampling a predetermined number of points of the objective variable groups;
an evaluation value calculation step of converting a vector whose dimension is a number of objective variables included in the objective variable groups and whose elements are values of the respective objective variables into a scalar through a predetermined operation, thereby calculating an evaluation value of an objective variable group at each sampling point;
an acquisition function evaluation step of receiving the design parameter group as input and outputting an acquisition function evaluation value related to improvement of the evaluation value using a predetermined acquisition function based on a distribution of the evaluation value at each sampling point;
a design parameter group acquisition step of acquiring at least one design parameter group by optimization of the acquisition function evaluation value; and
an output step of outputting the design parameter group acquired by the design parameter group acquisition step.

10. A design aid program for causing a computer to function as a design aid device for obtaining a plurality of design parameters improving a plurality of feature items indicating features of a product, a partly finished product, a semifinished product, a part, or a trial product to be applied to a method of optimizing a design parameter by repeating determination of a design parameter and manufacture of a product, a partly finished product, a semifinished product, a part, or a trial product based on the determined design parameter in design of a product, a partly finished product, a semifinished product, a part, or a trial product manufactured based on a design parameter group including a plurality of design parameters, the design aid program causing the computer to realize:

a data acquisition function of acquiring a plurality of pieces of performance data including the design parameter group and an observation value of each of the plurality of feature items with regard to the manufactured product, partly finished product, semifinished product, part, or trial product;

a model construction function of constructing, based on the performance data, a predictive model predicting an observation value of the feature item serving as an objective variable as a probability distribution or an approximation or alternative index thereof based on the design parameter group;

a sampling function of setting a plurality of objective variable groups sampled from a multidimensional probability distribution of observation values obtained from each predictive model as one sampling point, and sampling a predetermined number of points of the objective variable groups;

an evaluation value calculation function of converting a vector whose dimension is a number of objective variables included in the objective variable groups and whose elements are values of the respective objective variables into a scalar through a predetermined operation, thereby calculating an evaluation value of an objective variable group at each sampling point;

an acquisition function evaluation function of receiving the design parameter group as input and outputting an acquisition function evaluation value related to improvement of the evaluation value using a predetermined acquisition function based on a distribution of the evaluation value at each sampling point;

a design parameter group acquisition function of acquiring at least one design parameter group by optimization of the acquisition function evaluation value; and

an output function of outputting the design parameter group acquired by the design parameter group acquisition function.

# Fig.1

**Fig.2**

DESIGN AID DEVICE ~10

PROCESSOR ~101

DATA ACQUISITION UNIT ~11

MODEL CONSTRUCTION UNIT ~12

SAMPLING UNIT ~13

EVALUATION VALUE CALCULATION UNIT ~14

ACQUISITION FUNCTION EVALUATION UNIT ~15

DESIGN PARAMETER GROUP ACQUISITION UNIT ~16

OUTPUT UNIT ~17

DESIGN PARAMETER STORAGE UNIT ~21

OBSERVATION VALUE STORAGE UNIT ~22

EP 4 318 337 A1

# Fig.3

10

101 — PROCESSOR

102 — MAIN STORAGE DEVICE

103 — AUXILIARY STORAGE DEVICE

104 — COMMUNICATION CONTROL DEVICE

105 — INPUT DEVICE

106 — OUTPUT DEVICE

Fig.4

| DESIGN PARAMETERS IN FIRST TO (T - 1)TH MATERIAL MANUFACTURES | DESIGN PARAMETER GROUP | | | |
|---|---|---|---|---|
| | MIXING AMOUNT OF RAW MATERIAL A (d = 1) | MIXING AMOUNT OF RAW MATERIAL B (d = 2) | . . . | DESIGN PARAMETER D (d = D) |
| $x_1$ | . . . | . . . | . . . | . . . |
| $x_2$ | . . . | . . . | . . . | . . . |
| $x_3$ | . . . | . . . | . . . | . . . |
| $\vdots$ | . . . | . . . | . . . | . . . |
| $x_{T-1}$ | . . . | . . . | . . . | . . . |

EP 4 318 337 A1

# Fig.5

| NUMBER OF OBSERVATIONS | OBSERVATION VALUE $y_{k,t}$ OF FEATURE ITEM (1 to K) | | | |
|---|---|---|---|---|
| | GLASS TRANSITION TEMPERATURE (k = 1) | ADHESIVE STRENGTH (k = 2) | . . . | FEATURE ITEM K (k = K) |
| 1 | $y_{1,1}$ | $y_{2,1}$ | . . . | $y_{K,1}$ |
| 2 | $y_{1,2}$ | $y_{2,2}$ | . . . | $y_{K,2}$ |
| 3 | $y_{1,3}$ | $y_{2,3}$ | . . . | $y_{K,3}$ |
| : | : | : | : | : |
| T-1 | $y_{1,T-1}$ | $y_{2,T-1}$ | . . . | $y_{K,T-1}$ |

## Fig.6

```
                    ┌─────────┐
                    │  START  │
                    └────┬────┘
                         │
      ┌──────────────────┤
      │                  ▼
      │     ┌──────────────────────────┐
      │     │   ACQUIRE DESIGN         │── S1
      │     │   PARAMETER GROUP        │
      │     └────────────┬─────────────┘
      │                  │
      │     ┌────────────▼─────────────┐
      │     │ MATERIAL MANUFACTURE (TEST) │── S2
      │     └────────────┬─────────────┘
      │                  │
      │     ┌────────────▼─────────────┐
      │     │   ACQUIRE OBSERVATION    │── S3
      │     │   VALUE OF FEATURE ITEM  │
      │     └────────────┬─────────────┘
      │                  │         S4
      │                  ▼        ╱
      │           ◇───────────────◇       YES
      │          ╱ IS PREDETERMINED ╲──────────┐
      │          ╲ TERMINATION CONDITION ╱      │
      │           ◇   SATISFIED?  ◇             │
      │                  │                      │
      │                 NO                      │
      │                  │                      │
      │     ┌────────────▼─────────────┐        │
      │     │▌ DESIGN AID PROCESSING ▐ │── S5    │
      │     └────────────┬─────────────┘        │
      │                  │                      │
      └──────────────────┘                      │
                                                │
                    ┌─────────┐                 │
                    │   END   │◄────────────────┘
                    └─────────┘
```

# Fig.7

```
        ( DESIGN AID PROCESSING )
                     │
  ┌──────────────────────────────────┐
  │  ACQUIRE PERFORMANCE DATA         │
  │  INCLUDING PAIR OF DESIGN         │ ～S11
  │  PARAMETER GROUP AND OBSERVATION  │
  │  VALUE OF EACH FEATURE ITEM       │
  └──────────────────────────────────┘
                     │
  ┌──────────────────────────────────┐
  │  CONSTRUCT PREDICTIVE MODEL       │ ～S12
  └──────────────────────────────────┘
                     │
  ┌──────────────────────────────────┐
  │  SAMPLE PREDETERMINED NUMBER OF   │
  │  POINTS OF OBJECTIVE VARIABLE     │ ～S13
  │  GROUPS BASED ON PREDICTIVE MODEL │
  └──────────────────────────────────┘
                     │
  ┌──────────────────────────────────┐
  │  CALCULATE EVALUATION VALUE AT    │
  │  EACH SAMPLING POINT BY           │
  │  CONVERTING VECTOR WHOSE ELEMENTS │ ～S14
  │  ARE VALUES OF RESPECTIVE         │
  │  OBJECTIVE VARIABLES OF OBJECTIVE │
  │  VARIABLE GROUP INTO SCALAR       │
  └──────────────────────────────────┘
                     │
  ┌──────────────────────────────────┐
  │  OUTPUT ACQUISITION FUNCTION      │
  │  EVALUATION VALUE BASED ON        │ ～S15
  │  DISTRIBUTION OF EVALUATION VALUES│
  └──────────────────────────────────┘
                     │
  ┌──────────────────────────────────┐
  │  ACQUIRE DESIGN PARAMETER GROUP   │
  │  BY OPTIMIZATION OF ACQUISITION   │ ～S16
  │  FUNCTION EVALUATION VALUE        │
  └──────────────────────────────────┘
                     │
  ┌──────────────────────────────────┐
  │  OUTPUT ACQUIRED DESIGN           │ ～S17
  │  PARAMETER GROUP                  │
  └──────────────────────────────────┘
                     │
                 ( END )
```

# Fig.8

RECORDING MEDIUM — M1

DESIGN AID PROGRAM — P1

MAIN MODULE — m10

DATA ACQUISITION MODULE — m11

MODEL CONSTRUCTION MODULE — m12

SAMPLING MODULE — m13

EVALUATION VALUE
CALCULATION MODULE — m14

ACQUISITION FUNCTION
EVALUATION MODULE — m15

DESIGN PARAMETER GROUP
ACQUISITION MODULE — m16

OUTPUT MODULE — m17

## INTERNATIONAL SEARCH REPORT

| International application No. |
| --- |
| **PCT/JP2022/018312** |

| A. | CLASSIFICATION OF SUBJECT MATTER |
| --- | --- |

*G06N 20/00*(2019.01)i; *G06F 30/20*(2020.01)i; *G06F 30/27*(2020.01)i; *G06F 111/06*(2020.01)n
FI:   G06F30/20; G06F30/27; G06N20/00; G06F111:06

According to International Patent Classification (IPC) or to both national classification and IPC

| B. | FIELDS SEARCHED |
| --- | --- |

Minimum documentation searched (classification system followed by classification symbols)

G06N20/00; G06F30/20; G06F30/27; G06F111/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 2021-38344 A (NAT INST MATERIALS SCIENCE) 11 March 2021 (2021-03-11) paragraphs [0019]-[0063], fig. 1-8 | 1-10 |
| A | JP 2020-144799 A (FUJITSU LTD) 10 September 2020 (2020-09-10) paragraphs [0013]-[0116], fig. 1-8 | 1-10 |
| A | JP 2020-30683 A (YOKOHAMA RUBBER CO LTD) 27 February 2020 (2020-02-27) paragraphs [0020]-[0043], fig. 1-6 | 1-10 |
| A | JP 2020-71827 A (SHOWA DENKO KK) 07 May 2020 (2020-05-07) paragraph [0035] | 1-10 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **07 July 2022** | **19 July 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2022/018312**

| Patent document cited in search report | | | Publication date (day/month/year) | Patent family member(s) | | | Publication date (day/month/year) |
|---|---|---|---|---|---|---|---|
| JP | 2021-38344 | A | 11 March 2021 | (Family: none) | | | |
| JP | 2020-144799 | A | 10 September 2020 | US paragraphs [0025]-[0113], fig. 1-8 | 2020/0285690 | A1 | |
| | | | | EP | 3706029 | A1 | |
| | | | | CN | 111666708 | A | |
| JP | 2020-30683 | A | 27 February 2020 | (Family: none) | | | |
| JP | 2020-71827 | A | 07 May 2020 | US paragraph [0046] | 2020/0142951 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020052737 A